# EUROPÄISCHE PATENTSCHRIFT

(11) **EP 2 533 727 B1**
(45) Veröffentlichungstag und Bekanntmachung des Hinweises auf die Patenterteilung: **17.08.2016**
(21) Anmeldenummer: 11701195.7
(22) Anmeldetag: 11.01.2011
(51) Int. Cl.: A61B 17/88, B01F 13/00, B29B 7/24, B01F 11/00, B01F 15/02, A61F 2/46

(54) **MISCHVORRICHTUNG FÜR KNOCHENZEMENT SOWIE VERFAHREN ZUM MISCHEN VON KNOCHENZEMENT**
MIXING DEVICE FOR BONE CEMENT AND METHOD FOR MIXING BONE CEMENT
DISPOSITIF MÉLANGEUR POUR CIMENT OSSEUX AINSI QUE PROCÉDÉ POUR MÉLANGER DU CIMENT OSSEUX

(30) Priorität: 11.01.2010 DE 102010004342
(43) Veröffentlichungstag der Anmeldung: 19.12.2012
(73) Patentinhaber: AAP Biomaterials GmbH, 64807 Dieburg (DE)
(72) Erfinder: DE VRIES, Jan, Albert, 7021 HH Zelhem (NL); LOMAN, Wout, R., 7218 AH Almen (NL)
(74) Vertreter: Herden, Andreas F.
(86) Internationale Anmeldenummer: PCT/EP2011/000066
(87) Internationale Veröffentlichungsnummer: WO 2011/083095

(56) Entgegenhaltungen:
- WO-A1-99/22854
- WO-A1-2007/000631
- DE-A1- 19 532 015
- US-A1- 2003 155 381

## Beschreibung

### Gebiet der Erfindung

Die Erfindung betrifft eine Mischvorrichtung für Knochenzement.

### Hintergrund der Erfindung

Mischvorrichtungen für Knochenzement sind bekannt.
Eine derartige Mischvorrichtung zeigt beispielsweise die deutsche Patentanmeldung DE 10 2007 041 666 A1 der Anmelderin.

Beim Anmischen von Knochenzement wird ein Pulver, insbesondere ein Acrylat mit einem flüssigen Monomer vermischt. Nach dem Vermischen der Bestandteile kommt es zu einer Vernetzungsreaktion, bei der sich der Knochenzement zunehmend verfestigt. Der angemischte, aber noch nicht verfestigte Knochenzement wird beispielsweise in Markkanäle appliziert, in die sodann eine Prothese eingesetzt wird. Zur Vermeidung von Blasenbildungen oder zur Vermeidung, dass schädliche Gase in die Umwelt gelangen, gibt es Vakuum-Mischsysteme, bei denen am Mischbehälter ein Vakuumschlauch angebracht wird.

Derartige bekannte Vakuum-Mischsysteme haben den Nachteil, dass eine Pumpe erforderlich ist, um das das Vakuum zu erzeugen. Weiter bestehen beim Anmischen von Knochenzement hohe Anforderungen an die Sterilität. Nachteilig bei bekannten Mischsystemen ist, dass bereits während des Mischens Luft in den Mischbehälter eintritt.

Bekannte Knochenzement-Mischsysteme sind zudem in vielen Fällen umständlich zu bedienen und es besteht aufgrund der Vielzahl von erforderlichen Verfahrensschritten beim Anmischen die Gefahr, dass der Anwender Fehler macht, insbesondere dass das Verhältnis zwischen Monomer und Knochenzementpulver nicht richtig eingestellt wird.

Das Dokument WO 2007/000631 A1 zeigte eine Mischvorrichtung mit einer Kammer, die geöffnet wird, um eine Monomer in einem Mischraum gelangen zu lassen.

### Aufgabe der Erfindung

Der Erfindung liegt demgegenüber die Aufgabe zugrunde, eine möglichst einfach zu bedienende Knochenzement-Mischvorrichtung zur Verfügung zu stellen.

Weiter ist es eine Aufgabe der Erfindung, die Gefahr von Verunreinigungen des Knochenzements während des Mischvorgangs zu reduzieren.

### Zusammenfassung der Erfindung

Die Aufgabe der Erfindung wird bereits durch eine Mischvorrichtung für Knochenzement nach Anspruch 1 gelöst.

Bevorzugte Ausführungsformen und Weiterbildungen der Erfindung sind den Unteransprüchen zu entnehmen.

Die Erfindung kann eine Mischvorrichtung für Knochenzement vorsehen, welche ein Gehäuse mit einem Mischraum umfasst, in dem Monomer und Pulver mischbar sind. Der Mischraum ist als Zylinder ausgebildet und es ist mittels eines in dem Zylinder bewegbaren Kolbens ein Vakuum erzeugbar.

Das Vakuum wird somit direkt im Mischraum erzeugt und es ist daher keine Vakuumpumpe erforderlich. Die Ausbildung der Mischkammer als Zylinder, in welchem der Kolben bewegt wird, ermöglicht eine besonders einfache Ausbildung einer derartigen Mischvorrichtung mit wenigen Teilen, welche vorzugsweise als Einweg-Mischvorrichtung ausgebildet ist.

Es versteht sich, dass unter einem Vakuum nicht nur ein Druck von nahezu 0 bar, sondern ein Unterdruck verstanden wird.

Bei einer bevorzugten Ausführungsform ist der Hub des Kolbens derart bemessen ist, das ein definierter Restdruck zwischen 50 und 300 mbar, vorzugsweise zwischen 100 und 150 mbar erzeugt wird. Dies ist beispielsweise durch ein definiertes Leervolumen möglich, welches nicht über die Bewegung des Kolbens evakuiert wird. Durch den definierten Restdruck wird verhindert, dass sich im Monomer Gasblasen aufgrund des Erreichens des Siedepunktes durch die Druckherabsetzung bilden.

Zylinder und Kolben können einen nahezu beliebigen Querschnitt haben. Insbesondere ist ein kreisförmiger Querschnitt vorgesehen.

Vorzugsweise ist der Kolben manuell betätigbar, so dass auf einen Antriebsmotor verzichtet werden kann.

Gemäß einer Ausführungsform ist der Kolben mittels einer Gewindespindel im Mischraum bewegbar.

Es ist insbesondere eine Gewindespindel vorgesehen, welche eine mit dem Kolben verbundene Gewindestange aufweist, an welcher ein an den Mischraum angrenzender Drehgriff angreift. Der Drehgriff ist drehbar an dem Gehäuse der Mischvorrichtung angeordnet und weist eine Gewindemutter auf, die mit der Gewindestange korrespondiert. Die Gewindemutter kann auf sehr einfache Art und Weise ausgebildet sein, bei Verwendung geeigneter Kunststoffe reicht bereits ein Kunststoffschlitz, welcher mit den Flanken des Gewindes der Gewindestange korrespondiert. Dreht nun der Benutzer am Drehgriff in die vorgegebene Drehrichtung, so wird die Drehbewegung in eine Linearbewegung umgesetzt, welche den Kolben nach hinten bewegt und in dem verschlossenen Mischraum ein Vakuum erzeugt. Das Vakuum verhindert das Austreten von Gasen und verbessert die Vermischung der Komponenten im Mischraum.

Über die Steigung des Gewindes der Gewindestange kann das Übersetzungsverhältnis zwischen der Drehbewegung und der Linearbewegung bestimmt werden. Über die Steigung der Gewindestange kann so zum Einen das maximale Drehmoment und zum Anderen die Anzahl der erforderlichen Drehungen

bestimmt werden. Als besonders geeignet erwiesen hat sich eine Steigung von 0,5 bis 4 cm, besonders bevorzugt von 1,5 bis 2,5 cm. Die Gewindestange hat somit eine recht große Steigung, wodurch der Kolben mit nur wenigen Drehbewegungen in seine Endposition bewegbar ist.

Es ist auch denkbar, das Gehäuse des Mischraumes als Gewindespindel auszugestalten. Insbesondere können sich die Gewindegänge auf der Außenseite eines zylindrischen Gehäuses befinden.

Bei einer alternativen Ausführungsform der Erfindung ist der Kolben über eine vorgespannte Feder bewegbar. So kann beispielsweise der Kolben über eine Feder unter Vorspannung stehen und der Anwender entriegelt eine Entriegelungseinrichtung, welche die Feder freigibt. So lässt sich das Vakuum mit einem einzigen Handgriff erzeugen. Nachteilig an dieser Ausführungsform ist allerdings der etwas höhere Aufwand, sowie die Tatsache, dass der Kolben nicht über denselben Mechanismus in die Gegenrichtung bewegbar ist.

Bei einer Weiterbildung der Erfindung ist auf der dem Kolbenboden gegenüberliegenden Seite des Gehäuses ein bewegbares Mischpaddel angeordnet. Bei dieser Ausführungsform der Erfindung ist somit an dem vorzugsweise zylindrisch ausgebildeten Gehäuse auf einer Seite ein Mischpaddel und auf der anderen Seite der Kolben angeordnet.

Das Mischpaddel ist bei einer bevorzugten Ausführungsform der Erfindung über eine im Gehäuse geführte Stange mit einem Handhabungsgriff verbunden. Mit dem Handhabungsgriff kann das Mischpaddel in dem zylindrisch ausgebildeten Mischraum hin und her bewegt und/oder gedreht werden und so die Bestandteile des Knochenzements vermischen.

Gemäß der Erfindung ist die Stange zumindest abschnittsweise als ein mit dem Mischraum verbundenes Rohr ausgebildet und weist zumindest eine Sollbruchstelle auf.
So kann die Stange, mittels der das Mischpaddel bewegt wird, als eine Art Entnahmeschlauch benutzt werden. Dazu wird der Griff an der Sollbruchstelle von der restlichen Stange abgetrennt und es wird so eine Öffnung freigegeben. Bei einer Weiterbildung der Erfindung weist die Stange zumindest zwei voneinander beabstandete Sollbruchstellen auf. So lässt sich eine Art Entnahmeschlauch bereitstellen, welcher sich, je nachdem, wo die Stange auseinandergebrochen wird, auf zumindest zwei verschiedene Längen einstellen lässt. Abhängig von der jeweiligen Verwendung des Knochenzementes kann der Entnahmeschlauch so auf eine optimale Länge eingestellt werden.
Um ein besonders steriles und einfach zu bedienendes Applikationssystem bereitzustellen, ist bei einer bevorzugten Ausführungsform der Erfindung der Mischraum bereits mit Knochenzementpulver vorgefüllt.

Gemäß der Erfindung ist die Mischvorrichtung sowohl mit Knochenzement als auch mit einem Monomer vorgefüllt und hermetisch verschlossen.

Durch die Erfindung kann so eine Mischvorrichtung bereitgestellt werden, welche bis zur Applikation (zum Beispiel beim Abbrechen der zuvor beschriebenen Stange) vollständig verschlossen ist. Die Gefahr von Verunreinigungen durch die Raumluft wird so erheblich reduziert. Gleichzeitig besteht nicht die Gefahr, dass das Verhältnis von Knochenzementpulver und Monomer falsch eingestellt wird.

Bei einer Weiterbildung der Erfindung ist der Kolben zur Erzeugung des Vakuums zum Auspressen des Knochenzements in eine zweite Richtung bewegbar ausgebildet. Vorzugsweise erfolgt die Bewegung des Kolbens in die zweite Richtung ebenfalls über denselben Drehgriff, welcher zum Erzeugen des Vakuums verwendet wurde. Nach Anmischen des Knochenzements kann so der Kolben, der zuvor zur Erzeugung des Vakuums verwendet wurde, zum Auspressen des Knochenzements aus dem Mischraum benutzt werden.

Es ist bei einer derartigen Ausgestaltung nicht notwendig, die Mischvorrichtung mit weiteren Bauteilen zu verbinden, wie beispielsweise einer Pistole zum Auspressen einer als Kartusche ausgebildeten Mischvorrichtung.

Bei einer Weiterbildung der Erfindung weist der Mischraum eine Skalierung auf, so dass der Anwender erkennen kann, wie viel Knochenzement er appliziert hat.

Die Erfindung betrifft eine Mischvorrichtung für Knochenzement gemäß Anspruch 1, insbesondere eine Mischvorrichtung mit einem oder mehrerer zuvor beschriebener Merkmale.

Die Mischvorrichtung umfasst ein Gehäuse mit einem Mischraum, in welchem Monomer und Pulver mischbar sind, wobei im Mischraum ein Mischpaddel angeordnet ist, welches über eine im Gehäuse geführte Stange bewegbar ist.

Die Stange ist zumindest teilweise als Rohr ausgebildet, um nach dem Anmischen des Knochenzements als eine Art Entnahmeschlauch verwendet werden zu können.

Gemäß der Erfindung ist das Monomer in der Stange angeordnet. Das Volumen der Stange kann so zur Speicherung des Monomers genutzt werden. Die Handhabbarkeit der Mischvorrichtung wird erheblich verbessert, da diese bereits mit der notwendigen Menge Monomer vorgefüllt ist. Weiter ist eine sehr sterile Ausbildung der Mischvorrichtung sichergestellt.

Bei einer Weiterbildung der Erfindung ist das Monomer in einem Monomerbehälter angeordnet, welcher in den als Rohr ausgebildeten Abschnitt der Stange eingeführt ist.

Es ist so auf einfache Weise möglich, für den gesonderten Monomerbehälter ein Material zu verwenden, welches dauerhaft gegenüber dem Monomer beständig ist. Insbesondere ist ein mit einer Diffusionssperrschicht versehener Kunststoff oder ein Metall, insbesondere Aluminium, vorgesehen.

Insbesondere kann das Monomer in einem in die Stange eingeführten Einlegeschlauch angeordnet sein. Es kann sich dabei um einen länglichen Beutel aus flexiblem Material handeln, insbesondere aus einer Folie mit einer Diffusionssperrschicht.

Auch kann der Monomerbehälter Aluminiumfolie umfassen, insbesondere als Teil eines Schichtverbundes.

Bei einer Weiterbildung der Erfindung weist die Stange zum Bewegen des Mischpaddels einen Handhabungsgriff und eine Sollbruchstelle auf, wobei nach dem Abbrechen des Handhabungsgriffes der Monomerbehälter aus der Stange entfernbar ist.

Insbesondere ist vorgesehen, dass der Monomerbehälter als eine Art Röhrchen ausgebildet ist, welches - ohne entsprechende Sollbruchstelle - mit dem Handhabungsgriff verbunden ist. Nach dem Abbrechen des Griffes wird so der entleerte Monomerbehälter zusammen mit dem Handhabungsgriff aus dem als Rohr ausgebildeten Abschnitt der Stange herausgezogen. Beim Applizieren des Knochenzements sind so die Bestandteile des Monomerbehälters nicht mehr im Wege, die ansonsten das Austreten des zähflüssigen Knochenzements behindern könnten.

Bei einer Weiterbildung der Erfindung sind im Mischraum Mittel zum Einstechen des Monomerbehälters angeordnet.

Insbesondere ist vorgesehen, dass eine Spitze am Boden eines gegenüberliegenden Kolbens angeordnet ist. Beim erstmaligen Herunterdrücken des Mischpaddels kommt so die Spitze mit der Stirnseite des Monomerbehälters in Kontakt und der Monomerbehälter wird durch die Spitze aufgeschnitten oder eingedrückt, so dass das Monomer freigegeben wird.

Bei einer bevorzugten Ausführungsform der Erfindung ist der Monomerbehälter teilweise mit einem Gas, insbesondere mit Luft gefüllt. Der Monomerbehälter kann so vollständig verschlossen ausgebildet sein und nach dem Öffnen des Monomerbehälters wird das Monomer aufgrund der Expansion des Gasvolumens aus dem Monomerbehälter verdrängt. Bei geeigneter Abstimmung des Gasvolumens auf den Unterdruck, der vor dem Mischen im Mischraum vorhanden ist, genügt bereits die Expansion des Gases aufgrund des Druckunterschiedes. Alternativ oder in Kombination kann das Monomer auch mit einem Treibmittel versetzt sein.

Ein Verfahren zum Anmischen von Knochenzement, insbesondere mittels einer zuvor beschriebenen Mischvorrichtung wird beschrieben.

Zunächst wird mittels eines im Mischraum geführten Kolbens ein Vakuum erzeugt.
Über die Betätigung eines Mischpaddels über eine im Gehäuse der Mischvorrichtung geführte Stange wird ein an oder in der Stange angeordneter Monomerbehälter beim erstmaligen Betätigen eingestochen oder eingeschnitten und das Monomer tritt in einen mit Knochenzementpulver gefüllten Mischraum ein.

Durch weitere Betätigung des Mischpaddels über einen an der Stange angeordneten Handhabungsgriff wird der Knochenzement vermischt.

Nach dem Anmischen des Knochenzements wird der Handhabungsgriff abgebrochen, wodurch ein rohrförmiger Abschnitt der Stange einen Kanal nach außen freigibt.

Sodann wird der Kolben in Gegenrichtung bewegt, wodurch der Knochenzement durch den rohrförmigen Abschnitt der Stange austritt.

Das Verfahren ermöglicht ein besonders einfaches Anmischen von Knochenzement mit wenigen Handgriffen. Des Weiteren kann das Verfahren in einer hermetisch verschlossenen Mischvorrichtung ausgeführt werden, so dass der Knochenzement erst nach Abbrechen der Stange mit der Umgebungsluft in Kontakt kommt.

Vorzugsweise wird das Mischpaddel vor dem Abbrechen des Handhabungsgriffs in eine oberste Position bewegt, so dass beim Ausdrücken des Knochenzements dieser nicht an dem Mischpaddel vorbeigedrückt werden muss.

Das Monomer wird bei einer Weiterbildung der Erfindung durch ein im Monomerbehälter angeordnetes Gasvolumen aus dem Monomerbehälter in den Mischraum verdrängt.

Vorzugsweise wird der Monomerbehälter nach Entleerung zusammen mit dem abgebrochenen Handhabungsgriff aus der Stange gezogen.

### Kurzbeschreibung der Zeichnungen

Die Erfindung soll im Folgenden bezugnehmend auf Fig. 1 bis Fig. 9 anhand schematisch dargestellter Ausführungsbeispiele näher erläutert werden.
- Fig. 1: zeigt eine schematische Ansicht einer Mischvorrichtung
- Fig. 2: zeigt die in Fig. 1 dargestellte Mischvorrichtung in einer teilweise aufgeschnittenen Ansicht
- Fig. 3: ist eine Detaildarstellung eines Kolbens mit Gewindestange
- Fig. 4: zeigt eine Stange zur Bewegung des Mischpaddels
- Fig. 5: zeigt eine Ausführungsform eines Mischpaddels
- Fig. 6 bis Fig. 8: zeigen alternative Ausführungsvarianten eines Mischpaddels
- Fig. 9: zeigt ein Monomerbehältnis.

### Detaillierte Beschreibung der Zeichnungen

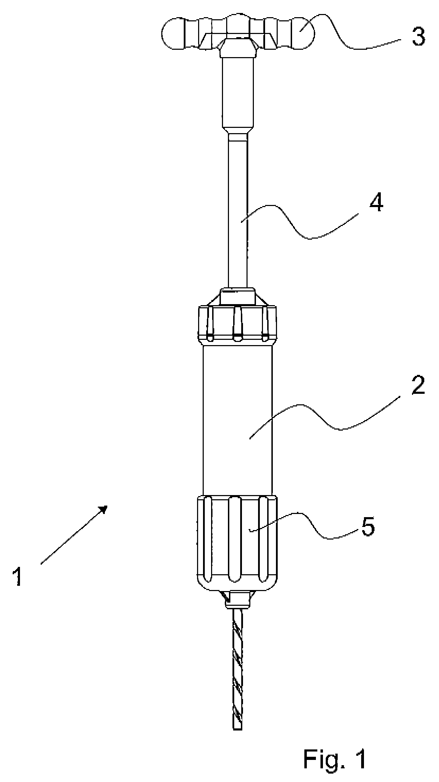

Bezugnehmend auf Fig. 1 sollen die wesentlichen Bestandteile einer Mischvorrichtung 1 für Knochenzement anhand eines schematisch dargestellten Ausführungsbeispiels näher erläutert werden.

Die Mischvorrichtung 1 umfasst ein im Wesentlichen zylinderförmig ausgebildetes Gehäuse 2, in welchem der Mischraum zum Anmischen des Knochenzements (nicht dargestellt) angeordnet ist.

Auf der Oberseite ist ein Handhabungsgriff 3 zu erkennen, der über eine Stange 4, welche im Gehäuse 2 geführt ist, mit einem Mischpaddel (nicht dargestellt) verbunden ist.

Auf der Unterseite ist ein Drehgriff 5 angeordnet.

Zum Anmischen und Applizieren des Knochenzements dreht der Benutzer (nicht dargestellt) zunächst den Drehgriff 5 in diesem Ausführungsbeispiel entgegen des Uhrzeigersinns. Nach Erreichen einer Endstellung des Drehgriffs 5 betätigt der Benutzer über den Handhabungsgriff 3 das Mischpaddel und bewegt es von oben nach unten, um den Knochenzement anzumischen. Bei erstmaliger Betätigung wird der Monomerbehälter (nicht dargestellt) automatisch geöffnet, so dass sich durch weiteres Bewegen des Handhabungsgriffs 3 der Knochenzement mischt.

Sodann bricht der Benutzer den Handhabungsgriff 3 ab.

Der verbleibende Abschnitt der Stange 4 ist als eine Art Rohr beziehungsweise Entnahmeschlauch ausgebildet

Sodann dreht der Benutzer den Drehgriff 5 in die entgegen gesetzte Richtung und der Knochenzement tritt aus der Stange 4 aus und kann appliziert werden.

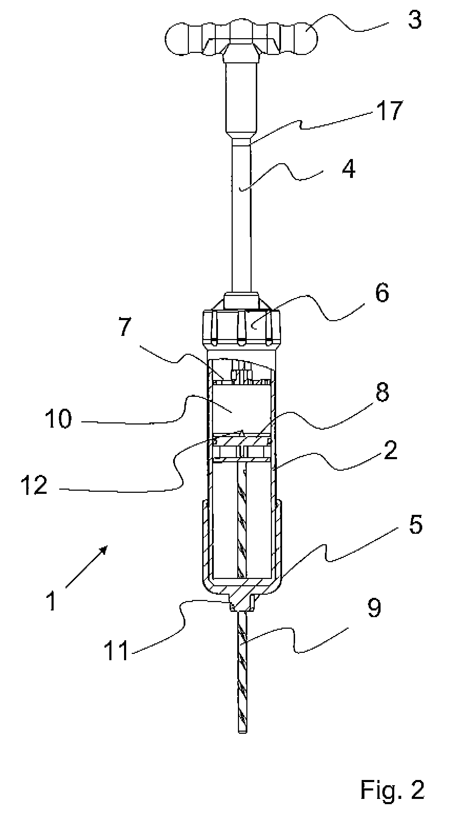

Fig. 2 zeigt eine teilweise aufgeschnittene Ansicht der in Fig. 1 dargestellten Mischvorrichtung 1. Zu erkennen ist insbesondere der Mischraum 10, welcher mit Knochenzementpulver (nicht dargestellt) vorgefüllt sein kann. Der Mischraum 10 ist als Zylinder ausgebildet, in welchem ein mit einer Gewindestange 9 verbundener Kolben 8 geführt ist.

Der Drehgriff 5 ist drehbar an dem Gehäuse 2 angeordnet. Der Drehgriff 5 umschließt in diesem Ausführungsbeispiel den unteren Teil des Gehäuses, wodurch eine besonders große Grifffläche bei nahezu unveränderten Abmessungen bereitgestellt werden kann.

Unten umfasst der Drehgriff 5 eine Spindelmutter, welche mit der Gewindestange 9 zusammenwirkt. Drehgriff 5 und Gewindestange 9 bilden so eine Gewindespindel, mittels der die rotatorische Bewegung des Drehgriffs 5 in eine translatorische Bewegung der Gewindestange und damit des Kolbens 8 umsetzbar ist.

So kann zunächst durch Zurückziehen des Kolbens 8 ein Vakuum im Mischraum 10 erzeugt werden.

Gegenüber des Kolbens ist auf der anderen Seite des Gehäuses 2 mittels der Stange 4 ein Mischpaddel 7 geführt, welches mit dem Handhabungsgriff 3 auf und ab bewegt werden kann. Bei erstmaliger Betätigung des Mischpaddels 7 stößt das Mischpaddel 7 gegen den Kolben 8, welcher eine Spitze 12 aufweist.

Die Spitze 12, welche am Boden des Kolbens 8 angeordnet ist, öffnet dabei ein mit dem Monomer gefülltes und in der Stange 4 angeordnetes Behältnis (nicht dargestellt), wodurch das Monomer in den Mischraum 10 eintritt.

Durch weitere Betätigung des Mischpaddels 7 werden Knochenzementpulver und Monomer zu Knochenzement vermischt.

Anschließend wird vom Benutzer der Handhabungsgriff 3 ganz nach oben gezogen und der Handhabungsgriff 3 an der an der Stange angeordneten Sollbruchstelle 17 abgebrochen. Die Stange 4 ist zumindest bis zur Sollbruchstelle 17 als Rohr ausgebildet und dient nach Abbrechen des Handhabungsgriffs 3 als Entnahmeschlauch.

Durch Drehen des Drehgriffes 5 in die Gegenrichtung bewegt sich der Kolben 8 nach oben und treibt den angemischten Knochenzement durch den als Rohr ausgebildeten Abschnitt der Stange 4 nach oben.

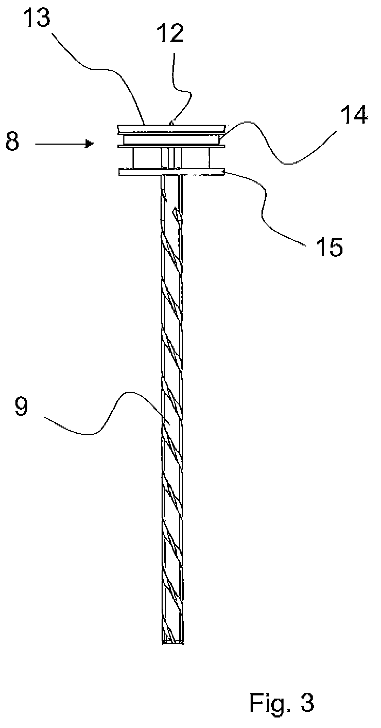

Fig. 3 zeigt eine Darstellung des Kolbens 8 und der Gewindestange 9.

Kolben 8 und Gewindestange 9 können als einstückiges Bauteil aus Kunststoff ausgebildet sein.

Der Kolben 8 weist am Kolbenboden 13 eine Spitze 12 auf, welche bei einer bevorzugten Ausführungsform der Erfindung als eingelassenes Metallteil ausgebildet ist.

Der Kolben 8 umfasst eine Nut 14, welche beispielsweise der Aufnahme einer Elastomerdichtung dienen kann.

Weiter umfasst der Kolben eine beabstandete Führungsplatte 15, wodurch die Gefahr einer Verkantung des Kolbens 8 reduziert wird.

Die Gewindestange 9 hat ein relativ steiles Gewinde, welches in diesem Ausführungsbeispiel eine Steigung von 1,5 bis 2,5 cm aufweist. So kann der Kolben 8 mit nur wenigen Drehbewegungen im Mischraum hoch und runter bewegt werden. Gleichzeitig kann durch geeignete Steigung des Gewindes der Gewindestange 9 gewährleistet werden, dass der Benutzer merkt, wenn sich der Knochenzement bereits zu stark verfestigt hat. Sodann ist das Hochbewegen des Kolbens derart erschwert, dass dies beim Drehen des Drehgriffes (5 in Fig. 1) erkennbar ist.

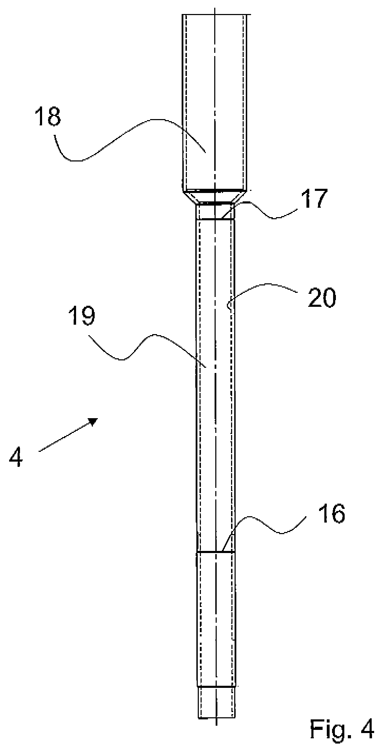

Fig. 4 zeigt eine Detaildarstellung der Stange 4, mit welcher das Mischpaddel (nicht dargestellt) bewegt wird.

An der Stange 4 wird am unteren Ende das Mischpaddel und am oberen Ende der Handhabungsgriff angebracht.

Die Stange 4 ist als Rohr ausgebildet, in welches ein Monomerbehälter eingeschoben werden kann.

Die Position des Monomerbehälters wird in diesem Ausführungsbeispiel durch die gestrichelte Linie 20 symbolisiert.

In diesem Ausführungsbeispiel hat der untere Abschnitt 19 der Stange einen kleineren Durchmesser als der obere Abschnitt 18 der Stange. An dem sich so zwischen den beiden Abschnitten ergebenden Kragen kann beispielsweise das Monomerbehältnis zur Anlage kommen.

Die rohrförmig ausgebildete Stange 4 weist eine untere Sollbruchstelle 16 und eine obere Sollbruchstelle 17 auf. So kann die Stange 4, welche zugleich als Entnahmeschlauch dient, je nach Applikation eine unterschiedliche Länge aufweisen. Das in der Stange 4 angeordnete Monomerbehältnis weist dagegen keine Sollbruchstelle auf, sondern ist fest mit dem Handhabungsgriff 3 (nicht dargestellt) verbunden. Nach Abbrechen das Handhabungsgriffs an der Sollbruchstelle 16 oder 17 wird das Monomerbehältnis aus der Stange 4 herausgezogen und die Reste des Monomerbehältnisses behindern nicht das Austreten des angemischten Knochenzementes.

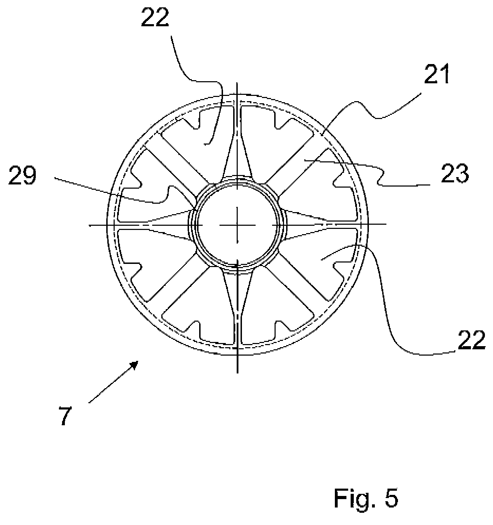

Fig. 5 zeigt schematisch dargestellt ein Mischpaddel 7.

Das Mischpaddel kann eine nahezu beliebige Form annehmen. In diesem Ausführungsbeispiel umfasst das Mischpaddel einen Ring 21, welcher über Stege 23 mit einem Innenring 29 verbunden ist. Der Innenring 29 wird auf der Betätigungsstange (4 in Fig. 2) befestigt. Zwischen den Stegen sind so Aussparungen 22 gebildet, durch welche der Knochenzement an den Stegen 22 vorbei hin und her strömen und so gemischt wird.

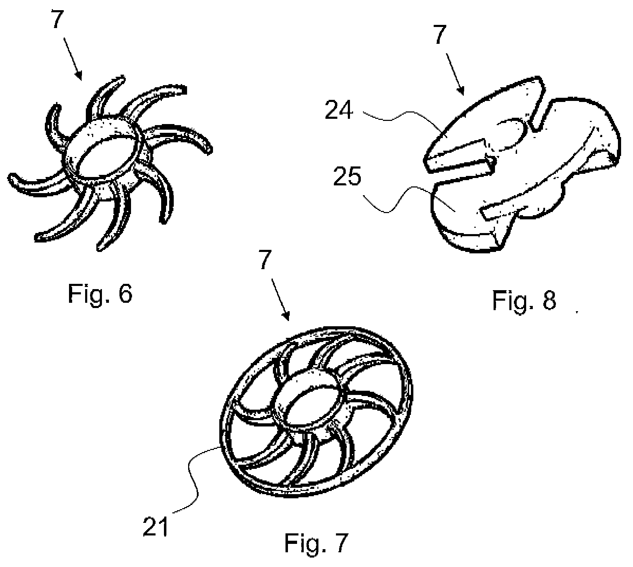

Fig. 6 bis Fig. 8 zeigen alternative Ausführungsvarianten eines Mischpaddels.

Das in Fig. 6 dargestellte Mischpaddel weist schräggestellte Flügel auf, um eine turbulente Strömung und damit eine bessere Durchmischung zu erreichen.

Ebenso ist das in Fig. 7 dargestellte Mischpaddel 7 ausgebildet, wobei hier zusätzlich noch ein Außenring vorhanden ist, der verhindert, dass inhomogen gemischte Reste sich an der Wand des Mischraums bilden.

Fig. 8 zeigt eine weitere Ausführungsform eines Mischpaddels 7, welches eine obere 24 und eine untere Platte umfasst. Der Knochenzement kann durch die zwischen den Platten gebildeten Zwischenräumen hindurchströmen. Diese Ausführungsform der Erfindung ermöglicht vor allem ein schnelles Anmischen des Knochenzements ohne jegliche Drehbewegung.

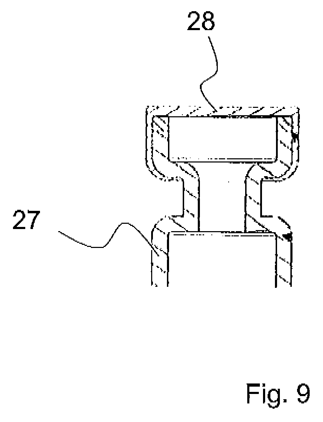

Fig. 9 zeigt eine Detaildarstellung eines Monomerbehälters 27, wobei in dieser Detaildarstellung nur der Kopf des Monomerbehälters 27 gezeigt ist.

Die Wände des Behälters 27 können aus Kunststoff oder aus Aluminium bestehen. Der Monomerbehälter 27 ist mit einem Deckel 28 verschlossen. Es kann sich dabei beispielsweise um einen aufgeschraubten Deckel oder, wie in diesem Ausführungsbeispiel, um einen aufgepressten Aluminiumdeckel oder einen Kunststoffdeckel, zum Beispiel aus PTFE, handeln, welcher mit einer geeigneten Abdichtung auf dem Monomerbehälter 27 aufsitzt. Der Deckel 28 kann zur Entnahme des Monomers mit einem spitzen Gegenstand, wie der in Fig. 3 gezeigten Spitze am Kolben eingestochen werden, wodurch das Monomer austritt. Um das Austreten des Monomers aus dem ansonsten geschlossenen Monomerbehälters 27 zu ermöglichen, ist der Monomerbehälter 27 teilweise mit einem Gasvolumen gefüllt, welches aufgrund des Druckunterschiedes bei anliegendem Vakuum das Monomer verdrängt (nicht dargestellt).

Die Erfindung ermöglicht ein besonders einfaches und sicheres Anmischen von Knochenzement.
Es versteht sich, dass die Erfindung nicht auf eine Kombination vorstehend beschriebener Merkmale beschränkt ist, sondern dass der Fachmann sämtliche Merkmale, soweit dies sinnvoll ist, in beliebiger Kombination verwenden wird, gemäß der Ansprüche 1 bis 11.

### Bezugszeichenliste

- 1: Mischvorrichtung
- 2: Gehäuse
- 3: Handhabungsgriff
- 4: Stange
- 5: Drehgriff
- 6: oberer Gehäuseteil
- 7: Mischpaddel
- 8: Kolben
- 9: Gewindestange
- 10: Mischraum
- 11: Spindelmutter
- 12: Spitze
- 13: Kolbenboden
- 14: Nut
- 15: Führungsplatte
- 16: Sollbruchstelle
- 17: Sollbruchstelle
- 18: oberer Abschnitt
- 19: unterer Abschnitt
- 20: gestrichelte Linie
- 21: Ring
- 22: Aussparung
- 23: Steg
- 24: obere Platte
- 25: untere Platte
- 27: Monomerbehälter
- 28: Deckel
- 29: Innenring

## Patentansprüche

1. Mischvorrichtung (1) für Knochenzement, umfassend ein Gehäuse (2) mit einem Mischraum (10), in welchem Monomer und Pulver mischbar sind, wobei die Mischvorrichtung (1) sowohl mit einem Knochenzement als auch mit dem Monomer vorgefüllt und hermetisch verschlossen ist, und wobei im Mischraum (10) Mittel zum Einstechen eines Monomerbehälters (27) angeordnet sind, wobei im Mischraum (10) ein Mischpaddel (7) angeordnet ist,
wobei die Mischvorrichtung eine im Gehäuse (2) geführte Stange (4) umfasst, die zumindest abschnittsweise als Rohr ausgebildet ist, wobei das Monomer in der Stange (4) angeordnet ist,
**dadurch gekennzeichnet, dass** das im Mischraum (10) angeordnete Mischpaddel (7) über die im Gehäuse (2) geführte Stange (4) bewegbar ist.

2. Mischvorrichtung (1) für Knochenzement nach dem vorstehenden Anspruch, **dadurch gekennzeichnet, dass** das Monomer in einem in den als Rohr ausgebildeten Abschnitt der Stange (4) eingeführten Monomerbehälter angeordnet ist.

3. Mischvorrichtung (1) für Knochenzement nach einem der vorstehenden Ansprüche, **dadurch gekennzeichnet, dass** das Monomer in einem in die Stange (4) eingeführten Einlegeschlauch angeordnet ist.

4. Mischvorrichtung (1) für Knochenzement nach einem der vorstehenden Ansprüche, wobei der Mischraum (10) als Zylinder ausgebildet ist und mittels eines in dem Zylinder bewegbaren Kolbens (8) ein Vakuum in dem Mischraum erzeugbar ist.

5. Mischvorrichtung (1) für Knochenzement nach dem vorstehenden Anspruch, **dadurch gekennzeichnet, dass** der Kolben (8) mittels einer Gewindespindel im Mischraum bewegbar ist.

6. Mischvorrichtung (1) für Knochenzement nach dem vorstehenden Anspruch, **dadurch gekennzeichnet, dass** die Gewindespindel eine mit dem Kolben (8) verbundene Gewindestange (9) aufweist, welche über einen an den Mischraum (10) angrenzenden Drehgriff mit Spindelmutter (11) bewegbar ist.

7. Mischvorrichtung (1) für Knochenzement nach Anspruch 4, **dadurch gekennzeichnet, dass** der Kolben (8) über eine vorgespannte Feder bewegbar ist.

8. Mischvorrichtung (1) für Knochenzement nach einem der vorstehenden Ansprüche, **dadurch gekennzeichnet, dass** auf der dem Kolbenboden (13) gegenüber liegenden Seite des Gehäuses (2) ein bewegbares Mischpaddel (7) angeordnet ist.

9. Mischvorrichtung (1) für Knochenzement nach dem vorstehenden Anspruch, **dadurch gekennzeichnet, dass** die Stange (4) zumindest abschnittsweise als ein mit dem Mischraum (10) verbundenes Rohr ausgebildet ist und zumindest eine Sollbruchstelle (17) aufweist.

10. Mischvorrichtung (1) für Knochenzement nach einem der vorstehenden Ansprüche, **dadurch gekennzeichnet, dass** der Hub des Kolbens (4) derart bemessen ist, das ein Restdruck zwischen 50 und 300 mbar, vorzugsweise zwischen 100 und 150 mbar erzeugt wird.

11. Mischvorrichtung (1) für Knochenzement nach einem der vorstehenden Ansprüche, **dadurch gekennzeichnet, dass** der Kolben (4) zur Erzeugung eines Vakuums in eine erste Richtung bewegbar ist und zum Auspressen des Knochenzements in eine zweite Richtung bewegbar ist.

## Claims

1. Mixing device (1) for bone cement, comprising a housing (2) having a mixing chamber (10) in which a monomer and powder can be mixed, wherein the mixing device (1) is prefilled with a bone cement and with the monomer and is hermetically sealed, and wherein in the mixing chamber (10) means for penetrating a monomer container (27) are arranged, wherein in the mixing chamber (10) a mixing paddle (7) is arranged,
wherein the mixing device comprises a rod (4) which is guided in the housing (2) and is formed at least in sections as a tube, wherein the monomer is arranged in the rod (4),
**characterised in that** the mixing paddle (7) arranged in the mixing chamber (10) can be moved via the rod (4) guided in the housing (2).

2. Mixing device (1) for bone cement as claimed in the preceding claim, **characterised in that** the monomer is arranged in a monomer container inserted into the section of the rod (4) formed as a tube.

3. Mixing device (1) for bone cement as claimed in any one of the preceding claims, **characterised in that** the monomer is arranged in an insert hose introduced into the rod (4).

4. Mixing device (1) for bone cement as claimed in any one of the preceding claims, wherein the mixing chamber (10) is formed as a cylinder and a vacuum can be generated in the mixing chamber by means of a piston (8) which can move in the cylinder.

5. Mixing device (1) for bone cement as claimed in the preceding claim, **characterised in that** the piston (8) can be moved by means of a threaded spindle in the mixing chamber.

6. Mixing device (1) for bone cement as claimed in the preceding claim, **characterised in that** the threaded spindle comprises a threaded rod (9) which is connected to the piston (8) and can be moved via a rotary handle which adjoins the mixing chamber (10) and has a spindle nut (11).

7. Mixing device (1) for bone cement as claimed in claim 4, **characterised in that**.the piston (8) can be moved via a pre-stressed spring.

8. Mixing device (1) for bone cement as claimed in any one of the preceding claims, **characterised in that** a movable mixing paddle (7) is arranged on the side of the housing (2) lying opposite the piston crown (13).

9. Mixing device (1) for bone cement as claimed in the preceding claim, **characterised in that** the rod (4) is formed at least in sections as a tube connected to the mixing chamber (10) and comprises at least one predetermined breaking point (17).

10. Mixing device (1) for bone cement as claimed in any one of the preceding claims, **characterised in that** the stroke of the piston (4) is sized such that a residual pressure between 50 and 300 mbar, preferably between 100 and 150 mbar, is produced.

11. Mixing device (1) for bone cement as claimed in any one of the preceding claims, **characterised in that** the piston (4) can be moved in a first direction for generating a vacuum and can be moved in a second direction for extruding the bone cement.

## Revendications

1. Dispositif mélangeur (1) pour ciment osseux, comprenant un boîtier (2) comportant une chambre de mélange (10) dans laquelle le monomère et la poudre peuvent être mélangés, le dispositif mélangeur (1) étant pré-rempli à la fois d'un ciment osseux et du monomère et fermé hermétiquement, et des moyens permettant de percer un contenant à monomère (27) étant agencés dans la chambre de mélange (10), une pale mélangeuse (7) étant agencée dans la chambre de mélange (10),
le dispositif mélangeur comprenant une tige (4) guidée dans le boîtier (2), qui est réalisée au moins en partie sous la forme d'un tuyau, le monomère étant situé dans la tige (4), **caractérisé en ce que** la pale mélangeuse (7) agencée dans la chambre de mélange (10) peut être déplacée par l'intermédiaire de la tige (4) guidée dans le boîtier (2).

2. Dispositif mélangeur (1) pour ciment osseux selon la revendication précédente, **caractérisé en ce que** le monomère est situé dans un contenant à monomère introduit dans la section de la tige (4) réalisée sous la forme d'un tube.

3. Dispositif mélangeur (1) pour ciment osseux selon l'une quelconque des revendications précédentes, **caractérisé en ce que** le monomère est situé dans un tuyau d'insertion introduit dans la tige (4).

4. Dispositif mélangeur (1) pour ciment osseux selon l'une quelconque des revendications précédentes, dans lequel la chambre de mélange (10) est réalisée sous la forme d'un cylindre et un vide peut être produit dans la chambre de mélange au moyen d'un piston (8) mobile dans le cylindre.

5. Dispositif mélangeur (1) pour ciment osseux selon l'une quelconque des revendications précédentes, **caractérisé en ce que** le piston (8) est mobile dans la chambre de mélange au moyen d'une broche filetée.

6. Dispositif mélangeur (1) pour ciment osseux selon l'une quelconque des revendications précédentes, **caractérisé en ce que** la broche filetée comprend une tige filetée (9) reliée au piston (8), laquelle peut être déplacée avec l'écrou de broche (11) au moyen d'une poignée rotative adjacente à la chambre de mélange (10).

7. Dispositif mélangeur (1) pour ciment osseux selon la revendication 4, **caractérisé en ce que** le piston (8) peut être déplacé au moyen d'un ressort précontraint.

8. Dispositif mélangeur (1) pour ciment osseux selon l'une quelconque des revendications précédentes, **caractérisé en ce qu'**une pale mélangeuse (7) mobile est agencée sur le côté du boîtier (2) opposé au fond (13) du piston.

9. Dispositif mélangeur (1) pour ciment osseux selon l'une quelconque des revendications précédentes, **caractérisé en ce que** la tige (4) est réalisée au moins en partie sous la forme d'un tube relié à la chambre de mélange (10) et présente au moins un point de rupture (17).

10. Dispositif mélangeur (1) pour ciment osseux selon l'une quelconque des revendications précédentes, **caractérisé en ce que** la course du piston (4) est mesurée de telle manière qu'une pression résiduelle comprise entre 50 et 300 mbar, de préférence entre 100 et 150 mbar, est produite.

11. Dispositif mélangeur (1) pour ciment osseux selon l'une quelconque des revendications précédentes, **caractérisé en ce que** le piston (4) peut être déplacé dans une première direction pour produire un vide et peut être déplacé dans une deuxième direction pour exprimer le ciment osseux.
